# EUROPÄISCHE PATENTANMELDUNG

(11) **EP 0 668 034 A1**
(43) Veröffentlichungstag der Anmeldung: **23.08.1995**
(21) Anmeldenummer: 95101910.8
(22) Anmeldetag: 13.02.1995
(51) Int. Cl.: A45D 29/00, A61B 17/54

(54) **Hornhautentferner**

(30) Priorität: 14.02.1994 DE 9402258 U; 05.04.1994 DE 9405640 U
(71) Anmelder: Excellent Gesellschaft für feine Schneidwaren mbH, D-42657 Solingen (DE)
(72) Erfinder: Schoenrock, Arno Otfried, D-51105 Köln (DE); Hartmann, Gerd Harald, 42719 Solingen (DE)
(74) Vertreter: Grundmann, Dirk, Dr.

(57) **Zusammenfassung**

Die Erfindung betrifft einen Hornhautentferner mit einem Reibkörper und einer den Reibkörper halternden Handhabe. Um solcherart bekannte Hornhautentferner herstellungstechnisch und gebrauchsvorteilhaft weiterzubilden, ist vorgesehen, daß der Reibkörper (1, 11, 12) von einer technischen Keramik gebildet ist und zwei im wesentlichen parallele Reibflächen (5, 6) mit unterschiedlicher Rauhigkeit aufweist, wobei der Reibkörper (1, 11, 12) von einer schlaufenförmigen Einfassung (3) der Handhabe (2) gehalten wird, welcher der Handgriff (4) angeformt ist.

## Beschreibung

Die Erfindung betrifft einen Hornhautentferner mit einem Reibkörper und einer den Reibkörper halternden Handhabe.

Derartige Hornhautentferner sind im Stand der Technik bekannt. Der Reibkörper ist dabei reibenartig oder raspelartig ausgebildet. Es sind auch handhabungslose Hornhautentferner bekannt, bei denen die Reibfläche von einem Bimsstein-Material ausgebildet wird.

Der Erfindung liegt die Aufgabe zugrunde, einen bekannten Hornhautentferner herstellungstechnisch und gebrauchsvorteilhaft weiterzubilden.

Gelöst wird die Aufgabe durch die im Anspruch 1 angegebene Erfindung.

Die Unteransprüche stellen vorteilhafte Weiterbildungen der Erfindung dar.

Zufolge der erfinderischen Weiterbildung eines gattungsgemäßen Hornhautentferners ist eine einfache Herstellung der Vorrichtung gegeben. Der Reibkörper ist als Reibstein ausgebildet und wird von einer schlaufenförmigen Einfassung der Handhabe gehaltert. Der steinartige Reibkörper wird von einer technischen Keramik gebildet. Bevorzugt wird dabei eine Feinkeramik, deren Zusammensetzung Glas, Aluminiumoxid und Calcium aufweist. Die Feinkeramik kann aus einer Mischung aus im wesentlichen 50% Glas und 48% Aluminiumoxid und 2% Calcium bestehen. Ergänzende Stoffe können bei Bedarf zugefügt werden. Als Glas kommt bevorzugt recyceltes Glas, insbesondere grünes Glas in Betracht. Der Hornhautentferner weist zwei sich gegenüberliegende Reibflächen auf, welche jeweils unterschiedliche Rauhigkeit aufweisen. Die Rauhigkeit der Oberflächen, welche parallel zueinander liegen können, kann durch die Korngröße der Korundkristalle eingestellt werden, welche die technische Keramik ausbilden. Eine bevorzugte Weiterbildung der Erfindung sieht vor, daß die Reibkörper von nachbeschichteten Keramikplatten ausgebildet werden. Dazu werden Keramikplättchen vorgefertigt, welche die Form der Schlaufeninnenfläche aufweisen. In einem weiteren Bearbeitungsschritt werden diese Keramikplatten mit Korundkristallen beschichtet und danach erneut gebrannt. Die Brenntemperatur liegt dabei bei 350 _{°} Celsius. Der Vorteil bei diesem Herstellungsverfahren liegt darin, daß zunächst eine große Menge von gleichen Keramikplättchen vorproduziert werden kann. Wegen der nachfolgenden Beschichtung ist die Oberflächeneigenschaft dieser vorproduzierten Plättchen von untergeordneter Bedeutung. In dem zweiten Brennschritt wird dann die Oberflächenrauhigkeit der Plättchen eingestellt. Dies geschieht durch die Auswahl der Korngröße der Beschichtungskristalle. Bevorzugt liegt die Kristallgröße auf der rauhen Seite in einem Bereich von 0,3 bis 0,5 mm und auf der feinen Seite im Bereich von 0,05 bis 0,1 mm. Eine besonders bevorzugte Ausgestaltung der Erfindung sieht vor, daß der Reibkörper aus zwei Hälften besteht. Die von den beiden Hälften ausgebildeten Einzelreibkörper liegen parallel zueinander. Die Trennfläche zwischen den beiden Reibkörpern verläuft dabei parallel zu den beiden gegenüberliegenden Reibflächen. Der Vorteil dieser Ausgestaltung liegt darin, daß nunmehr jeder Reibkörper eine spezifische Rauhigkeit aufweist. Bei der Beschichtung der Keramikplättchen braucht für jeden Stein nur ein Beschichtungmaterial mit einer einzigen Korngröße verwendet werden. Beim Brennvorgang schmilzt das Glas und klebt die Korundkörper zusammen bzw. mit dem das Substrat bildende Keramikplättchen zusammen. Der Vorteil der Verwendung einer technischen Keramik für den Hornhautentferner besteht darin, daß die Reibfläche nicht an Qualität und Größe verliert. Die Reibfläche ist mit Wasser leicht reinigbar und deshalb hygienisch. Weiterhin lassen sich in einfacher Weise eine grobe und eine feine Seite einstellen. Es ist weiter vorgesehen, daß die feine Seite der Vorrichtung als Peeling- oder Massagegerät verwendet wird. Besonders vorteilhafte Wirkungen werden dann erzielt, wenn der den Reibkörper ausbildende Stein vor der Anwendung befeuchtet wird. Durch die schlaufenförmige Einfassung des Reibsteines ist es möglich, diesen mit zwei unterschiedlich rauhen Oberflächen auszugestalten, wobei beide Oberflächen einem einstückigen Reibkörper zugeordnet sind. Der Reibkörper kann von beiden Seiten benutzt werden. Er braucht nicht selbst angefaßt zu werden, dem Bediener steht hierfür vielmehr ein Griff zur Verfügung, welcher der Einfassung angeformt ist. Der Reibstein kann in der schlaufenförmigen Einfassung einliegen oder eingeklemmt sein. Die Befestigung kann entweder formschlüssig oder reibschlüssig sein. Hierzu ist die Einfassung elastisch. Zufolge der Rauhigkeit der Oberfläche des vorzugsweise elliptisch ausgestalteten Reibsteins ist eine reibschlüssige Halterung in einer vorzugsweise aus Kunststoff bestehenden Einfassung bevorzugt. Der Stein ist auch umwendbar. Der Stein kann aber auch in der Einfassung eingeklebt sein. Insbesondere durch die schlaufenartige Einfassung des Steines ist eine beidseitige Benutzung ermöglicht. Der aus einer technischen Keramik bestehende Reibstein ist so mit einer individuell bestimmbaren Oberflächenrauhigkeit reproduzierbar herstellbar. Es ist denkbar, daß die unterschiedliche Rauhigkeit der sich gegenüberliegenden Reibsteinoberflächen durch die Anordnung der Korundkristalle, welche den Reibsteinwerkstoff ausbilden, eingestellt werden kann. Weiter ist es möglich, durch eine Vielzahl von Kristallen, welche untereinander bei der Herstellung verschmolzen sein können, dreidimensionale Cluster auszubilden. Diese Cluster liegen benachbart und sind ihrerseits wieder miteinander verbunden (verschmolzen), so daß insgesamt ein poröser Werkstoff gebildet wird. Wird die unterschiedliche Rauhigkeit durch eine unterschiedlich dichte Anordnung dieser Cluster ausgebildet, so können bei der einen Seite des Reibsteines, welcher die geringe Rauhigkeit aufweist, die Cluster in dichter Anordnung nebeneinander liegen, so daß die Rauhigkeit im wesentlichen von der Größe der Kristalle ausgebildet wird, deren Korngröße wesentlich kleiner ist als die Größe der Cluster. Die rauhere Seite weist dagegen eine Oberfläche auf, bei der die Cluster mit größerem Abstand zueinander liegen, so daß dort die Rauhigkeit im wesentlichen durch die Korngröße der Cluster bestimmt wird.

Bei der besonders bevorzugte Ausführungsform, bei welcher zwei parallel zueinander angeordnete Reibkörper vorgesehen sind, kann vorgesehen sein, daß zwischen den Reibkörpern ein Luftspalt belassen ist. In diesen Zwischenraum ragen von der Schlaufe ausgehende Stege, welche die beiden Reibkörperhälften distanzieren. Die beiden Reibkörperhälften sind durch Klebstoff oder Kunstharzbrücken miteinander verbunden. Hierdurch ist eine gewisse Elastizität zwischen den beiden Reibkörpern gegeben. Die Stege können dabei einem umlaufenden Stützkragen angeformt sein, welcher der Innenwandung der Schlaufe angeformt ist und in den Zwischenraum zwischen den beiden Reibkörpern ragt.

Ein Ausführungsbeispiel der Erfindung wird anhand der beigefügten Zeichnungen nachfolgend erläutert. Es zeigen
Fig. 1 einen Hornhautentferner in der Ansicht,
Fig. 2 einen Hornhautentferner in der Draufsicht,
Fig. 3 einen Schnitt gemäß der Linie 111-111 in Fig. 2,
Fig. 4 eine perspektivische Darstellung des Hornhautentferners bei entnommenem Reibstein,
Fig. 5 ein zweites Ausführungsbeispiel der Erfindung in einer Darstellung gemäß Fig. 3, und
Fig. 6 eine Schnittdarstellung gemäß Fig. 4 der zweiten Ausführungsform.

Der Hornhautentferner besteht aus einer Handhabe 2, welche einen Handgriff 4 aufweist, welcher sich unter Ausbildung einer Abkröpfung 8 in Längsrichtung in eine Einfassung 3 erstreckt. Die Einfassung 3 bildet eine Schlaufe aus zur Einfassung des Reibsteins 1, 11, 12. Die Konturlinie der Innenfläche 3' der Schlaufe 3 hat dabei in etwa die Form der Außenkontur 1' des Reibsteins 1, 11, 12 und ist elliptisch. Die von der Innenkontur 3' umschlossene Fläche ist - wenn überhaupt - nur geringfügig kleiner als die Querschnittsfläche des Reibsteines, so daß der Reibstein zufolge seiner abrasiven Seitenoberfläche reibschlüssig in der elastisch nachgebbaren Einfassung 3 gehaltert ist.

Die Handhabe 2 ist bevorzugt aus Kunststoff gefertigt, so daß der Griff 4 und die Einfassung 3 materialeinheitlich als Spritzgußteil hergestellt werden können. An dem der Einfassung 3 gegenüberliegenden Ende des Handgriffes 4 ist eine Öffnung 7 vorgesehen, mit welcher der Hornhauthobel aufgehängt werden kann. Der Griff 4 weist darüber hinaus an seiner Oberfläche eine Riffelung 9 auf. Die Rillen der Riffelung sind dabei schräg zur Erstreckungsrichtung des Griffes 4 angeordnet. Sie befinden sich einerseits auf der Oberseite und andererseits gegenüberliegend auf der Unterseite des Griffes 4.

Der Reibstein 1, 11, 12 besteht aus einer technischen Keramik. Der Reibstein 1 hat in etwa die Form eines Zylinders, wobei die Querschnittsfläche des Zylinders elliptisch ist. Die Ellipse weist dabei eine Hälfte mit geringerer Krümmung und eine Hälfte mit größerer Krümmung auf. Hierdurch läuft die Ellipse einseitig keilförmig zu. Bevorzugt verläuft die keilförmige Richtung spitzenseitig in entgegengesetzte Richtung des Handgriffes 2.

Der Werkstoff des Reibsteines 1, 11, 12 besteht aus einer Vielzahl von mikroskopisch kleinen Korundkristallen. Diese Kristalle haben in etwa die Form und die Größe von feinen Sandkörnern. Die Rauhigkeit der Oberfläche 5, 6 der Reibkörper 1, 11, 12 kann durch die Korngröße der Korundkristalle eingestellt werden. Die feine Seite 5 weist dabei eine Korngröße von 0,05 bis 0,1 mm auf, die rauhe Seite 6 eine Korngröße von 0,3 bis 0,5 mm. Die Kristalle sind im Werkstoff zu größeren Klumpen oder Clustern 10 zusammengeschmolzen. Da die Cluster von einer Vielzahl von Kristallen ausgebildet werden, haben sie einen wesentlich größeren Durchmesser als die Kristalle selbst. Auch diese Cluster 10 sind im Reibstein 1 zusammengeschmolzen, so daß ein einheitlicher fester Körper gebildet ist, der zufolge der Abstände der Kristalle und der Abstände der Cluster eine poröse Struktur aufweist. Dabei sind Hohlräume verschiedenartiger Größe entstanden. Die kleinen Hohlräume entstehen durch die Beabstandung der einzelnen Korundkristalle, zwischen welchen der entsprechende Hohlraum ausgebildet ist. Die größeren Hohlräume entstehen durch die Beabstandung der Cluster 10.

Bei der Reibfläche 5, welche parallel ausgerichtet ist zur Reibfläche 6, können die Cluster 10 besonders eng nebeneinanderliegen, so daß die Hohlräume zwischen den benachbarten Clustern 10 minimal ist. Diese Seite 5 ist dann die Seite mit der geringen Rauhigkeit. Die gegenüberliegende Seite 6 kann größere Abstände zwischen den einzelnen Clustern 10 aufweisen, so daß diese Oberfläche eine größere Rauhigkeit aufweist.

Der Reibstein 1 kann sowohl in die schlaufenförmige Einfassung 3 eingeklemmt sein als aber auch in die schlaufenförmige Einfassung 3 eingeklebt sein. Erstere Ausgestaltung ermöglicht ein Umwenden des Reibsteins. Die schlaufenförmige Einfassung des zylinderförmigen Reibsteins 1 ermöglicht es aber insgesamt, daß der Reibstein von beiden Seiten benutzt werden kann, da die Halterung nur die Umfangsfläche braucht.

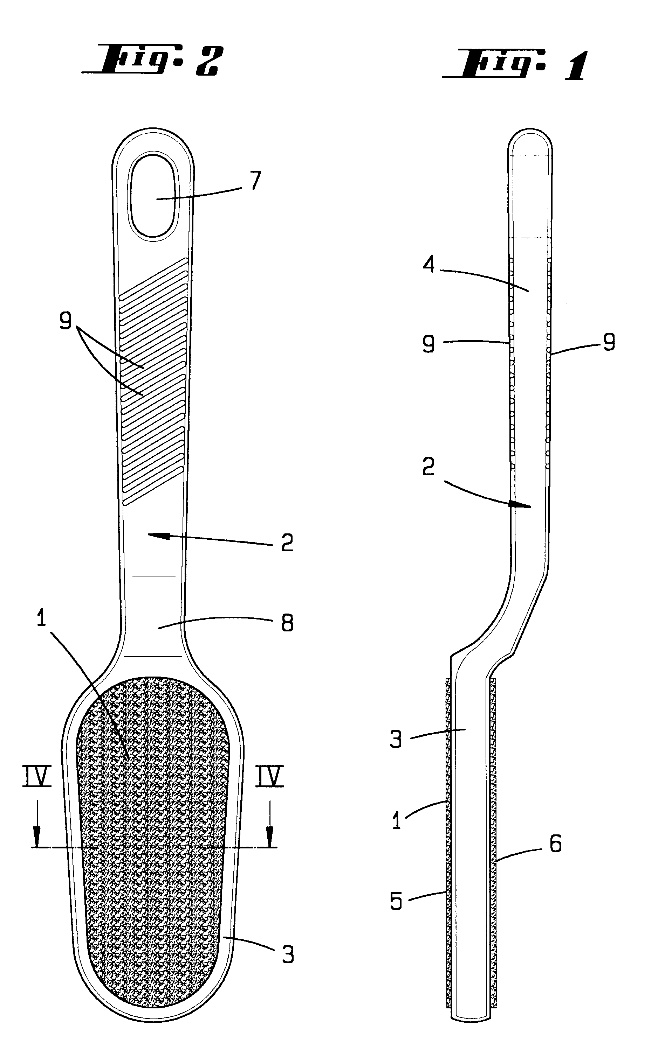

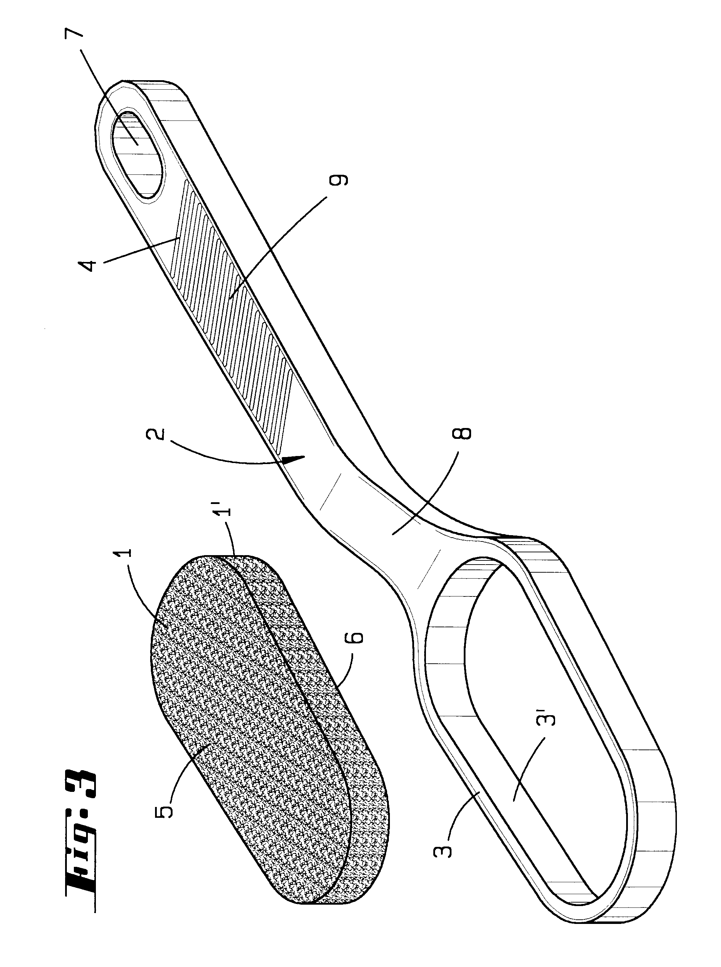

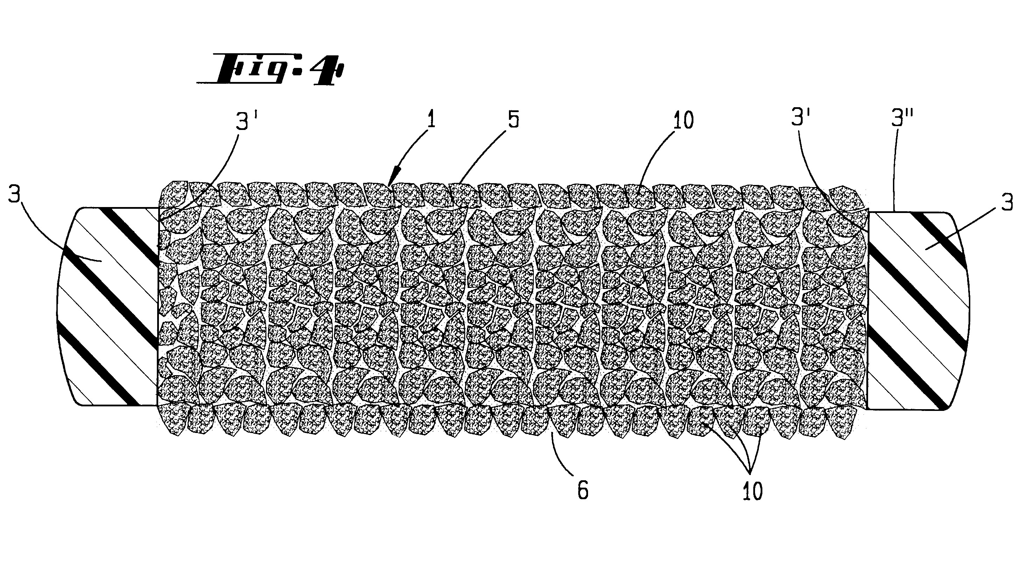

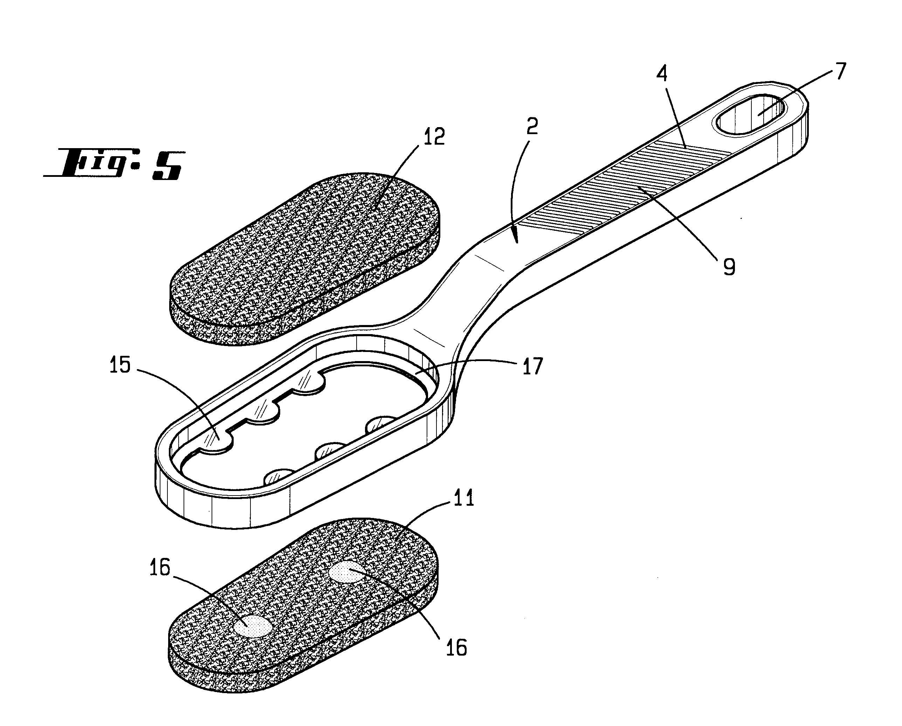

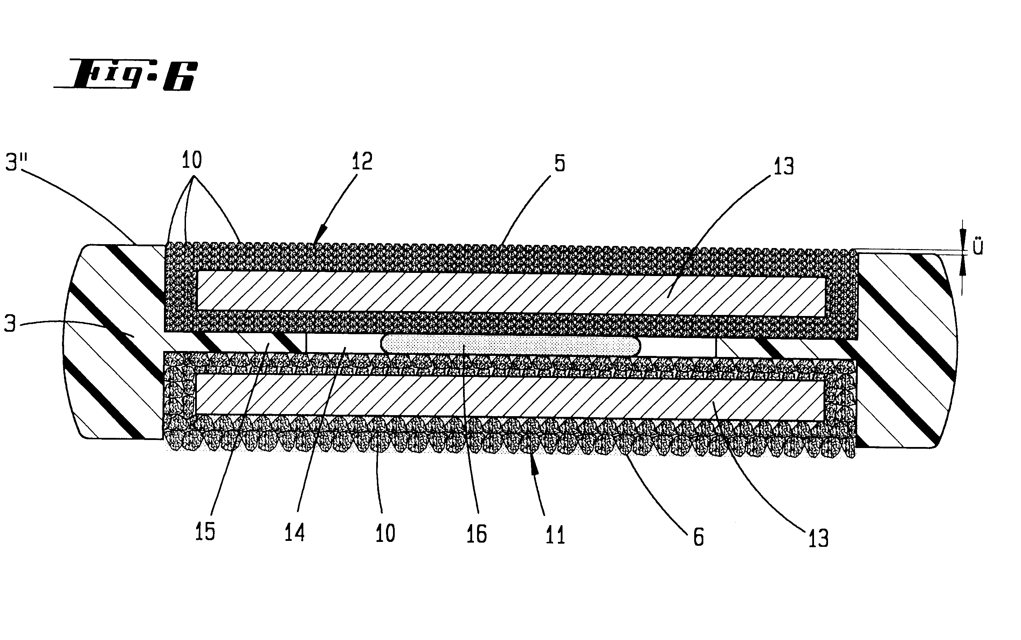

Während beim ersten Ausführungsbeispiel (Fig. 1-4) ein einzelner Reibkörper 1 Verwendung findet, sind beim zweiten Ausführungsbeispiel (Fig. 5 und 6) zwei Reibkörper 11, 12 vorgesehen. Anders als beim Reibkörper 1 des ersten Ausführungsbeispiels, welcher Oberflächen mit unterschiedlicher Rauhigkeit aufweist, haben die Reibkörper 11, 12 jeweils eine einheitliche Rauhigkeit. Dabei kann die Oberfläche 6 des Reibkörpers 11 rauher sein als die Oberfläche 5 des Reibkörpers 12. Die Rauhigkeit bei den Reibkörpern 11, 12 wird durch eine unterschiedliche Korngröße der Korundkristalle 10 eingestellt.

Zur Herstellung der Reibkörper 11, 12 wird zunächst ein Keramikplättchen 13 vorgefertigt, dessen Flächenerstreckung in etwa der Flächenerstreckung des Schlaufeninnenraumes der Schlaufe 3 entspricht. In einem zweiten Bearbeitungsschritt wird dieses Keramikplättchen 13 mit Korundkristallen 10 einer bestimmten Korngröße beschichtet. Es reicht aus, das entsprechende Keramikplättchen 13 nur einseitig mit einer bestimmten Korngröße zu beschichten.

Durch die Wahl der Korngröße, die für die feine Oberfläche 0,05 bis 0,1 mm entspricht und für die grobe Oberfläche 0,3 bis 0,5 mm, wird die Rauhigkeit der Reibkörper 11, 12 eingestellt, die Beschichtung besteht aus einer Feinkeramik und zwar 50% aus recyceltem, bevorzugt grünem Glas und weiteren 50% aus einer Mischung aus Aluminiumoxid und Calcium und weiteren Stoffen. Letztere Mischung weist einen Anteil von etwa 96% Aluminiumoxid und etwa 4% Calcium auf. Die zusätzliche Beschichtung wird bei einer Temperatur von 35 Celsius durchgeführt. Bei diesem Brennvorgang schmilzt das Glas und klebt in Matrix Korundkörner ein. In vorteilhafter Weise ist es durch den aus zwei nahezu identischen Hälften bestehenden Reibkörper 11, 12 möglich, eine Vielzahl von Keramikplättchen 13 vorzuproduzieren und diese dann individuell zu beschichten. Ein aus zwei Hälften bestehender Reibstein hat darüber hinaus den Vorteil, daß die beiden Hälften einen Spalt zwischen sich aufweisen und in der Schlaufe 3 aufgenommen werden können, so daß zwischen den Reibkörpern 11, 12 eine gewisse elastische Beweglichkeit entsteht.

Wie insbesondere der Fig. 6 zu entnehmen ist, ist zwischen den beiden Reibkörpern 11, 12 ein Spalt 14 vorgesehen, in welchen vom Rand der Schlaufe 3 ausgehende Stege 15 ragen, die einerseits die Reibkörper 11, 12 tragen und andererseits die Reibkörper 11, 12 distanzieren. Die Reibkörper 11, 12 sind durch eine Klebstoffschicht oder eine Kunstharzschicht 16 miteinander verbunden. Hierdurch entfällt eine Haftverbindung unmittelbar mit der Schlaufe 3. Der aus den Hälften 11, 12 gebildete Reibkörper sitzt so formschlüssig in der Schlaufe 3. Der Überstand Ü der Oberfläche 5, 6 gegenüber der Seitenfläche 3" der Schlaufe 3 betrifft etwa 0,5 mm. Die Stege 15 können einem umlaufenden Kragen 17 angeformt sein, welcher den Randbereich des Spaltes 14 ausfüllt. Die Vorrichtung ist auch als Nagelfeile verwendbar.

Die in der vorstehenden Beschreibung, der Zeichnung und den Ansprüchen offenbarten Merkmale der Erfindung können sowohl einzeln als auch in beliebiger Kombination für die Verwirklichung der Erfindung von Bedeutung sein. Alle offenbarten Merkmale sind erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen.

## Patentansprüche

1. Hornhautentferner mit einem Reibkörper und einer den Reibkörper halternden Handhabe, dadurch gekennzeichnet, daß der Reibkörper (1, 11, 12) von einer technischen Keramik gebildet ist und zwei im wesentlichen parallele Reibflächen (5, 6) mit unterschiedlicher Rauhigkeit aufweist.

2. Hornhautentferner nach Anspruch 1, dadurch gekennzeichnet, daß der Reibkörper (1, 11, 12) von einer schlaufenförmigen Einfassung (3) der Handhabe (2) gehalten wird, welcher der Handgriff (4) angeformt ist.

3. Hornhautentferner nach oder insbesondere nach einem oder mehreren der vorhergehenden Ansprüche, dadurch gekennzeichnet, daß der Reibkörper aus einer Feinkeramik besteht, deren Zusammensetzung Glas, Aluminiumoxid und Calcium aufweist.

4. Hornhautentferner nach oder insbesondere nach einem oder mehreren der vorhergehenden Ansprüche, dadurch gekennzeichnet, daß die Zusammensetzung im wesentlichen aus etwa 50% Glas, 48% Aluminiumoxid und 2% Calcium besteht.

5. Hornhautentferner nach oder insbesondere nach einem oder mehreren der vorhergehenden Ansprüche, dadurch gekennzeichnet, daß der Glasanteil von recyceltem, vorzugsweise grünem Glas gebildet wird.

6. Hornhautentferner nach oder insbesondere nach einem oder mehreren der vorhergehenden Ansprüche, dadurch gekennzeichnet, daß die Reibkörper aus vorgefertigten Keramikplatten (13) bestehen, die nach einer Beschichtung mit Korundkristallen (10) erneut gebrannt werden.

7. Hornhautentferner nach oder insbesondere nach einem oder mehreren der vorhergehenden Ansprüche, dadurch gekennzeichnet, daß die Rauhigkeit durch die Korngröße der Korundkristalle (10) einstellbar ist.

8. Hornhautentferner nach oder insbesondere nach einem oder mehreren der vorhergehenden Ansprüche, dadurch gekennzeichnet, daß die Kristallgröße für die rauhe Seite (6) im Bereich von 0,3 bis 0,5 mm und für die feine Seite (5) im Bereich von 0,05 bis 0,1 mm liegt.

9. Hornhautentferner nach oder insbesondere nach einem oder mehreren der vorhergehenden Ansprüche, dadurch gekennzeichnet, daß der Reibkörper aus zwei parallel zueinander liegenden Reibkörperhälften (11, 12) gebildet wird.

10. Hornhautentferner nach oder insbesondere nach einem oder mehreren der vorhergehenden Ansprüche, dadurch gekennzeichnet, daß die beiden Reibkörperhälften (11, 12) mit ihren Breitseiten verklebt sind und formschlüssig in der Schlaufe (3) gehalten sind.

11. Hornhautentferner nach oder insbesondere nach einem oder mehreren der vorhergehenden Ansprüche, dadurch gekennzeichnet, daß der Überstand (Ü) der Reibfläche (5,6) über dem Randbereich (3") der Halteschlaufe (3) etwa 0,5 mm beträgt.

12. Hornhautentferner nach oder insbesondere nach einem odermehreren der vorhergehenden Ansprüche, dadurch gekennzeichnet, daß die Klebeverbindung (16) zwischen den beiden Reibkörperhälften (11, 12) eine Trennfuge (14) ausbildet, in welche an der Schlaufe (3) angeformte Stege (15) ragen.

13. Hornhautentferner nach oder insbesondere nach einem oder mehreren der vorhergehenden Ansprüche, dadurch gekennzeichnet, daß der Reibkörper (1) umwendbar in der Einfassung einliegt oder eingeklemmt ist.

14. Hornhautentferner nach oder insbesondere nach einem oder mehreren der vorhergehenden Ansprüche, dadurch gekennzeichnet, daß der Reibkörper (1) in der Einfassung (3) eingeklebt ist.

15. Hornhautentferner nach oder insbesondere nach einem oder mehreren der vorhergehenden Ansprüche, dadurch gekennzeichnet, daß der Reibstein (1) aus einer Vielzahl von miteinander zu Clustern verbundenen, insbesondere verschmolzenen Korundkristallen besteht.

16. Hornhautentferner nach oder insbesondere nach einem oder mehreren der vorhergehenden Ansprüche, dadurch gekennzeichnet, daß die Korundkristalle dreidimensionale Cluster (10) ausbilden, welche miteinander verbunden bzw. verschmolzen sind.

17. Hornhautentferner nach oder insbesondere nach einem oder mehreren der vorhergehenden Ansprüche, dadurch gekennzeichnet, daß die eine Seite (5) des Reibsteins (1) mit geringerer Rauhigkeit eine dichtere Clusteranordnung aufweist als die gegenüberliegende Seite (6) mit größerer Rauhigkeit.
